## Europäisches Patentamt

## European Patent Office

## Office européen des brevets

(11) Veröffentlichungsnummer: **0 137 142**

**B1**

(12) # EUROPÄISCHE PATENTSCHRIFT

(45) Veröffentlichungstag der Patentschrift:
04.01.89

(21) Anmeldenummer: 84108160.7

(22) Anmeldetag: 12.07.84

(51) Int. Cl.⁴: **A 61 M 25/00,** A 61 M 16/04

(54) **Ballonkatheter, insbesondere Endotrachealkatheter, mit Konnektor.**

(30) Priorität: 16.07.83 DE 3325797
03.02.84 DE 3403681

(43) Veröffentlichungstag der Anmeldung:
17.04.85 Patentblatt 85/16

(45) Bekanntmachung des Hinweises auf die Patenterteilung:
04.01.89 Patentblatt 89/1

(84) Benannte Vertragsstaaten:
AT BE CH DE FR GB IT LI LU NL SE

(56) Entgegenhaltungen:
DE-A-2 327 231
DE-A-2 625 570
DE-B-2 745 899
DE-B-2 837 813
DE-C-823 181
US-A-2 693 191
US-A-4 020 849
US-A-4 198 984
US-A-4 222 384

(73) Patentinhaber: NATEC Institut für
naturwissenschaftlich- technische Dienste
GmbH, Behringstrasse 154, D-2000 Hamburg 50
(DE)

(72) Erfinder: Hammerschmidt, Winrich, Dr.,
Wachtelweg 35a, D-2000 Hamburg- Schenefeld
(DE)
Erfinder: Zumbruch, Gerhard, Engentwiete 8,
D-2000 Norderstedt 3 (DE)

(74) Vertreter: Schulmeyer, Karl- Heinz, Dr., Kieler
Strasse 59a, D-2087 Hasloh (DE)

EP 0 137 142 B1

## Beschreibung

Vorliegende Erfindung betrifft einen Ballonkatheter, insbesondere Endotrachealkatheter, mit einem Katheterrohr aus elastischem, gewebefreundlichem Material mit einem offenen proximalen Ende, das mit einem angepaßten Konnektor verbindbar ist, einer in der Nähe des distalen Rohrendes auf dem Katheterrohr angeordneten, mit ihm fest verbundenen, aufblasbaren Blockermanschette, einem in der Nähe des proximalen Rohrendes auf dem Katheterrohr angeordneten, mit ihm fest verbundenen, aufblasbaren Kontrollballon und ein oder mehreren Zuleitungsrohren, die die Blockermanschette mit dem Kontrollballon und mit einem Ansatzstutzen für eine Blockerspritze verbinden.

Ballonkatheter, bei denen Blockermanschette und Kontrollballon auf dem Katheterrohr angeordnet und über ein oder mehrere Zuleitungsrohre miteinander und mit einem Ansatzstutzen für die Blockerspritze zum Aufblasen von Blockermanschette und Kontrollballon verbunden sind, sind bereits bekannt und werden beispielsweise in DE-C-1 491 793, DE-A-1 925 852, DE-B-2 246 526, DE-B-2 426 344, DE-A-2 803 094 und DE-C-3 028 568 beschrieben. Sie haben sich in der Chirurgie gut eingeführt und werden in verschiedenen Formen vom Handel angeboten. Ihre Fertigung ist jedoch ziemlich aufwendig. Da man aus hygienischen Gründen, insbesondere zur Vermeidung von Infektionen im Krankenhaus, dem sogenannten Infektionshospitalismus, immer häufiger dazu übergeht, jeden Patienten nur noch mit fabrikneuen, sterilen Instrumenten zu behandeln, ist man bestrebt, auch die Ballonkatheter in zunehmendem Maße als Steril-Instrumente zum einmaligen Gebrauch bereitzustellen und einzusetzen.

Es hat sich jedoch gezeigt, daß die bisher üblichen Bauarten der Ballonkatheter für eine einmalige Verwendung außerordentlich kostspielig sind. So setzt sich beispielsweise ein Endotrachealkatheter herkömmlicher Bauart mit Blockermanschette im wesentlichen aus 6 verschiedenen Einzelteilen zusammen, nämlich dem Beatmungsrohr, der Blockermanschette, dem Kontrollballon, dem Belüftungsrohr als Verbindung zwischen Manschette und Kontrollballon, dem Konnektor und dem Ansatzstutzen am Kontrollballon für eine Blockerspritze. Andere Ballonkatheter sind in ähnlicher Weise zusammengesetzt. Die Teile müssen einzeln gefertigt und danach zusammengesetzt und gegebenenfalls fest miteinander verbunden werden. Die Herstellung ist daher sehr zeit- und arbeitsaufwendig.

Aus der DE-A-2 625 570 ist ein Katheter mit einem über ein Aufblaslumen aufblasbaren Manschettenballon bekannt, der über das Aufblaslumen mit einem Sicherheits- oder Signalballon in Verbindung steht, wobei beide Ballons auf dem Katheterrohr angeordnet sind.

Der zum Aufblasen der Manschette und des Signalballons erforderliche Füllstrom wird in der Weise geführt, daß zuerst die in der Nähe des distalen Rohrendes befindliche Manschette aufgeblasen wird. Dagegen soll der Signalballon erst aufgeblasen werden, wenn der Aufblasdruck in der Manschette einen vorbestimmten Druck erreicht hat. Das bedeutet, daß der Sicherheitsballon in Strömungsrichtung hinter dem Manschettenballon angebracht ist. Das soll den Vorteil haben, daß während des Aufblasvorganges der Sicherheitsballon keinem höheren Druck ausgesetzt ist als der Manschettenballon. Aus der Beschreibung und den Figuren der zitierten Offenlegungsschrift geht hervor, daß dieser bekannte Katheter einen komplizierten Aufbau besitzt und daher nur ziemlich aufwendig hergestellt werden kann.

Der in der US-A-3 543 759 beschriebene Katheter ist ähnlich aufgebaut, d. h. Manschetten- und Signalballon sind auf dem Katheterrohr so angeordnet, daß sich der Manschettenballon in der Nähe des distalen Rohrendes und der Signalballon in der Nähe des proximalen Rohrendes befinden. Beide Ballons sind durch ein Lumen miteinander und mit der Außenatmosphäre verbunden. Beim Aufblasen durchströmt der Füllstrom zunächst den Signalballon und dann erst den Manschettenballon. Die Konstruktion dieses bekannten Katheters ist ähnlich kompliziert wie die des Katheters aus der oben beschriebenen DE-A-2 625 570.

Aus der DE-B-2 837 813 ist bekannt, ein Katheterrohr zur Bildung und dichten Befestigung eines Ballons mit einer Mantelschicht aus thermoplastischem oder elastomerem Material durch Extrudieren zu ummanteln. Hierbei wird allerdings nur die Herstellung eines Ballons auf dem Katheterrohr beschrieben, d. h. es handelt sich um übliche Katheterformen, bei denen sich nur der Manschettenballon auf dem Katheterrohr befindet, während der Signal- oder Kontrollballon, wenn überhaupt vorhanden, auf dem seitlich aus dem oberen Bereich des Katheterrohres abzweigenden selbständigen Verbindungsschlauch angeordnet ist. Das Zuleitungsrohr, über das der Ballon gefüllt bzw. aufgeblasen werden kann, liegt in der Wand des Katheterrohres.

In der US-A-4 222 384 wird ein aus einer Reihe von einzelnen Teilen zusammengesetzter Katheter beschrieben. Die Katheterspitze wird zusammen mit dem Ballonteil in einem Stück geformt, daran schließt sich ein Innenkern mit längsgerichteten Rillen an, die auf der Außenoberfläche des Kerns gebildet sind. Weitere Teile des Katheters sind eine Hülse, die den Innenkern umgibt, sowie ein Fülltrichter am proximalen Ende, der einen Drainageteil und einen davon abgezweigten Verbindungsteil für die Zuleitungsrohre aufweist. Die genannten Teile müssen einzeln hergestellt und anschließend in einem ziemlich komplizierten Verfahren zusammengesetzt und miteinander verklebt werden.

Die vorerwähnten Katheter sind aufgrund ihrer relativ komplizierten Bauart nicht einfach zu fertigen und daher für den einmaligen Gebrauch in der Regel zu teuer. Es besteht daher ein dringender Bedarf nach Ballonkathetern, insbesondere Endotrachealkathetern, die so rationell hergestellt werden können, daß die Herstellungskosten einen Einmalgebrauch solcher Instrumente in wirtschaftlich vertretbarer Weise ermöglichen.

Vorliegender Erfindung liegt daher die Aufgabe zugrunde, Ballonkatheter, insbesondere Endotrachealkatheter, zu schaffen, die sich gegenüber den herkömmlichen Ballonkathetern mit Manschette und Kontrollballon durch eine wesentlich einfachere Konstruktion unterscheiden und die sich durch ein erheblich kostengünstigeres Verfahren herstellen lassen, so daß ihre Verwendung zum einmaligen Gebrauch auch vom wirtschaftlichen Standpunkt aus vertretbar erscheint.

Diese Aufgabe wird erfindungsgemäß gelöst durch einen Ballonkatheter, insbesondere Endotrachealkatheter, mit einem Katheterrohr aus elastischem, gewebefreundlichem Material mit einem offenen proximalen Ende, das mit einem angepaßten Konnektor verbindbar ist, einer in der Nähe des distalen Rohrendes auf dem Katheterrohr angeordneten, mit ihm fest verbundenen, aufblasbaren Blockermanschette, einem in der Nähe des proximalen Rohrendes auf dem Katheterrohr angeordneten, mit ihm fest verbundenen, aufblasbaren Kontrollballon und ein oder mehreren Zuleitungsrohren, die die Blockermanschette mit dem Kontrollballon und mit einem Ansatzstutzen für eine Blockerspritze verbinden. Der erfindungsgemäße Katheter ist dadurch gekennzeichnet, daß das Katheterrohr mit einem abgeschrägten, offenen distalen Ende über seine gesamte Länge in an sich bekannter Weise durch Aufextrudieren eines dünnwandigen Folienschlauches ummantelt ist, wobei dieser Folienschlauch in den für die Blockermanschette und den Kontrollballon vorgesehenen Bereichen lose auf der Außenwand des Katheterrohres aufliegt, während der Schlauch in den übrigen Bereichen fest und dicht mit der Außenwand des Katheterrohres verbunden ist, wobei das oder die Zuleitungsrohre, die sich über die gesamte Länge des Katheterrohres erstrecken und am distalen Rohrende dicht verschlossen sind, durch jeweils eine Rinne in der Außenwand des Katheterrohres und den das Katheterrohr dicht ummantelnden, die Rinne nach außen gas- und flüssigkeitsdicht abdeckenden Folienschlauch gebildet sind, und daß das Katheterrohr und das bzw. die Zuleitungsrohre am proximalen Ende mit dem Konnektor über eine oder mehrere Anschlußöffnungen und entsprechende Ansatzstutzen verbindbar sind.

Ein besonderer Vorteil des erfindungsgemäßen Ballonkatheters besteht in seiner einfachen Konstruktion, bei der nicht nur die Blockermanschette, sondern auch der Kontrollballon fest verbunden auf dem Katheterrohr angeordnet sind. Dadurch wird eine wesentliche Vereinfachung des Herstellungsverfahrens des Katheters bei gleicher oder sogar besserer praktischer Brauchbarkeit erzielt. An Einzelteilen fallen nur das Katheterrohr, das ein oder mehrere Rinnen in seiner Außenwand aufweist, und ein Folienschlauch an, der das Katheterrohr erfindungsgemäß über seine gesamte Länge gas- und flüssigkeitsdicht ummantelt und nur in den für die Blockermanschette und den Kontrollballon vorgesehenen Bereichen lose auf der Außenwand des Katheterrohres aufliegt. Dadurch sind Blockermanschette und Kontrollballon als dünnwandige, aufblasbare Teile des das Katheterrohr ummantelnden Folienschlauches in einem Stück ausgestaltet. Der Folienschlauch wird, gegebenenfalls gleichzeitig mit dem Katheterrohr, in geeigneter Weise durch ein Extrusionsverfahren hergestellt und so auf das Katheterrohr aufgebracht, daß er es eng anliegend ummantelt.

Der erfindungsgemäße Ballonkatheter setzt sich demnach nur noch aus 2 Einzelteilen zusammen. Es liegt auf der Hand, daß sich auf diese Weise die Fertigung wesentlich vereinfacht und der erfindungsgemäße Aufbau des Ballonkatheters eine Serienproduktion zuläßt, bei der das ummantelte Katheterrohr als "Endlosrohr" kontinuierlich, z. B. durch ein kombiniertes Schlauchextrusions- und Extrusionsblasverfahren, hergestellt und in bestimmten Abständen abgelängt werden kann. Die dadurch möglich werdende Massenproduktion erlaubt es, die erfindungsgemäßen Ballonkatheter in verhältnismäßig kurzer Zeit in großer Stückzahl herzustellen, so daß der Preis für das Einzelstück so niedrig kalkuliert werden kann, daß eine wirtschaftlich vertretbare Verwendung des erfindungsgemäßen Ballonkatheters für den einmaligen Gebrauch möglich wird.

Blockermanschette und Kontrollballon sind fest auf dem Katheterrohr angebracht und umhüllen das Katheterrohr über praktisch die gesamte Länge der Manschette bzw. des Ballons, wobei sie über die durch Rinne und abdeckenden Folienschlauch gebildeten ein oder mehreren Zuleitungsrohre, die im Bereich der Ballone als offene Rinnen vorliegen, aufgeblasen bzw. entlüftet werden können.

Die feste Verbindung des Folienschlauches mit dem Katheterrohr kann durch geeignete Maßnahmen bewirkt werden, deren Auswahl unter anderem vom Material abhängt, aus dem die zu verbindenden Teile bestehen. Vorzugsweise bestehen sowohl das Katheterrohr als auch der Folienschlauch, aus dem in den vorgesehenen Bereichen Blockermanschette und Kontrollballon gebildet werden, aus Gummi, insbesondere Weichgummi, oder einem synthetischen Polymeren, wie PVC, Hochdruckpolyethylen oder einem thermoplastischen Polyurethan oder aus hochpolymeren elastischen Organo-Silicium-Verbindungen. Die feste, gas- und flüssigkeitsdichte Verbindung des Folienschlauches mit dem Katheterrohr außer an den für die Blockermanschette und den Kontrollballon vorgesehenen Stellen erfolgt vorzugsweise durch Verkleben, Siegeln, Verschweißen und/oder Aufschrumpfen.

Mit dem erfindungsgemäßen Ballonkatheter ist ferner ein an die Erfordernisse des Ballonkatheters angepaßter Konnektor fest, gegebenenfalls aber lösbar verbunden.

Beim erfindungsgemäßen Ballonkatheter münden am proximalen Ende sowohl das Katheterrohr als auch das bzw. die mit dem Kontrollballon und der Blockermanschette in Verbindung stehenden ein oder mehreren Zuleitungsrohre. Der Konnektor muß daher so ausgebildet sein, daß er gleichzeitig sowohl die Verbindung des Katheterrohres mit dem gewünschten medizinischen Gerät, z. B. einem Beatmungs-, Narkose- oder Absaugegerät, als auch die Verbindung des Zuleitungsrohres mit einer geeigneten Luftzufuhrvorrichtung, z. B. einer eine dosierte Luftmenge abgebenden Luftspritze oder einer Luftpumpe, z. B. einer Blockerspritze, gewährleistet. Dies wird in einer erfindungsgemäß bevorzugten Ausführungsform des Ballonkatheters dadurch erreicht, daß der Katheter mit einem angepaßten Konnektor versehen ist, der einen weiten Rohrstutzen und einen engen Rohrstutzen aufweist, wobei der Übergang von dem weiten Rohrstutzen zu dem engen Rohrstutzen durch ein konisch geformtes Rohrstück gebildet wird, und ein weites Rohrstück enthält, das um den engen Rohrstutzen konzentrisch angeordnet und mit einem Ansatzrohrstück für eine Luftzufuhrleitung versehen ist, wobei das Ansatzrohrstück über einen ringförmigen Durchlaß mit dem zwischen der Innenwand des weiten Rohrstücks und der Außenwand des engen Rohrstutzens gebildeten hohlzylinderförmigen Raum verbunden ist und der Abstand zwischen der Innenwand des weiten Rohrstückes und der Außenwand des engen Rohrstutzens der Wandstärke des einzuführenden Katheterrohres so angepaßt ist, daß sich eine gasdichte Verbindung ergibt.

Dieser Konnektor kann aufgrund seiner vorteilhaften Konstruktion rasch und problemlos mit dem erfindungsgemäßen Ballonkatheter verbunden und verwendet werden. Der Konnektor besitzt den besonderen Vorteil, daß er auf das Katheterrohr mit dem in der Wand befindlichen und am Ende des Katheterrohres aus der Wand mündenden Zuleitungsrohr in jeder beliebigen Stellung paßt, so daß kein Verkanten oder Danebenstecken vorkommen kann. Wenn das proximale Ende des Katheterrohres über das Endstück des engen Rohrstutzens so weit aufgeschoben wird, daß sich das aufgeschobene Katheterrohr gasabdichtend zwischen dem weiten Rohrstück und dem engen Rohrstutzen befindet, dann bildet sich dadurch ein abgeschlossener hohlzylinderförmiger Luftraum zwischen dem Ende des aufgeschobenen Katheterrohres und dem Eintritt der durch das Ansatzrohrstück tretenden Luftzufuhrleitung in den hohlzylinderförmigen Raum. Die in diesem Raum befindliche Luft kann bei beliebiger Stellung des Konnektors zu dem Katheterrohr leicht in das in der Wand des Katheterrohres befindliche Zuleitungsrohr eintreten. Dadurch wird die Sicherheit in der Handhabung des Konnektors erheblich erhöht, ein Vorteil, der erst aufgrund der spezifischen, vorteilhaften und einfachen Konstruktion gemäß der Erfindung ermöglicht wird.

Nachfolgend wird die Erfindung am Beispiel einer Ausführungsform eines Endotrachealkatheters anhand von Zeichnungen näher erläutert. Es zeigen:

Figur 1    eine erfindungsgemäße Ausführungsform des Endotrachealkatheters in Seitenansicht;

Figur 2    einen Querschnitt durch den in Figur 1 gezeigten Endotrachealkatheter längs einer Linie 2 - 2;

Figur 3    eine perspektivische Ansicht einer Ausführungsform des an das Katheterrohr angepaßten Konnektors;

Figur 4    einen schematischen Längsschnitt durch die in Figur 3 dargestellte Ausführungsform des Konnektors;

Figur 5    die perspektivische Ansicht einer weiteren Ausführungsform des Konnektors und

Figur 6    einen schematischen Längsschnitt durch die in Figur 5 dargestellte Ausführungsform des Konnektors.

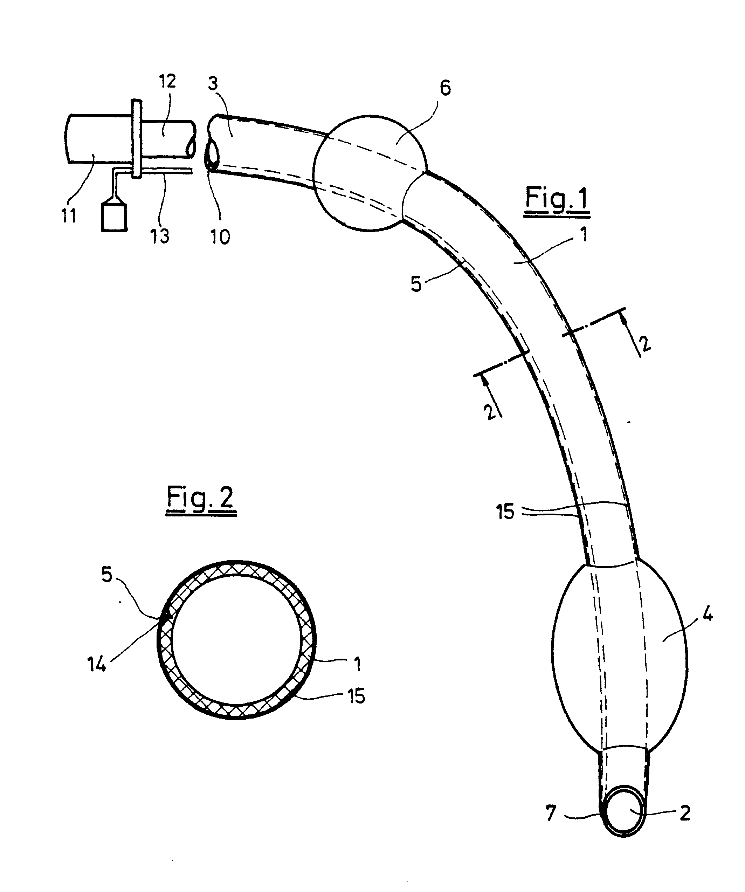

Der in Figur 1 dargestellte Endotrachealkatheter besteht aus einem Katheterrohr 1 aus elastischem, gewebefreundlichem Material mit einem abgeschrägten und abgerundeten offenen distalen Ende 2, das in die Luftröhre eines Patienten eingeführt wird, und einem offenen proximalen Ende 3, das mit einem Beatmungs- oder Narkosegerät über einen angepaßten Konnektor verbindbar ist.

Das Katheterrohr 1 ist über seine gesamte Länge außen von einem dünnwandigen Folienschlauch 15 fest und dicht ummantelt, bis auf die Bereiche, die für die Blockermanschette 4 und den Kontrollballon 6 vorgesehen sind. An diesen Stellen liegt der Folienschlauch 15 nur lose auf der Außenwand des Katheterrohres 1 auf. In einer bevorzugten Ausführungsform ist der Folienschlauch 15 in den für die Blockermanschette 4 und den Kontrollballon 6 vorgesehenen Bereichen aufgeweitet. Wie aus Fig. 1 hervorgeht, ist die aufblasbare Blockermanschette 4 in der Nähe des distalen Rohrendes 2 in einem Abstand davon auf dem Katheterrohr 1 in der Weise angeordnet, daß sie dieses Rohr hohlzylinderartig umgibt und mit ihm fest und gas- und flüssigkeitsdicht verbunden ist. In gleicher Weise ist in der Nähe des proximalen Rohrendes 3 in einem Abstand davon der aufblasbare Kontrollballon 6 auf dem Katheterrohr 1 so angeordnet, daß er das Katheterrohr 1 hohlzylinderartig umgibt und fest und gas- und flüssigkeitsdicht mit ihm verbunden ist.

Die Form und Größe von Blockermanschette 4 und Kontrollballon 6 sind nicht an die aus Fig. 1 ersichtliche Form und Größe gebunden; in der Regel ist jedoch die Blockermanschette 4 von länglicher Form und größer als der Kontrollballon 6.

Aus Fig. 1 in Verbindung mit Fig. 2 ist ersichtlich, daß in der Außenwand des Katheterrohres 1 ein oder mehrere Rinnen 14 vorgesehen sind (in den Figuren 1 und 2 sind einfachheitshalber jeweils nur eine Rinne 14 bzw. ein Zuleitungsrohr 5 dargestellt), die, wie aus Fig. 2 hervorgeht, von dem Folienschlauch 15, der das Katheterrohr 1 dicht und fest ummantelt, so abgedeckt werden, daß sich dadurch ein oder mehrere Zuleitungsrohre 5 bilden. Innerhalb des Bereichs der Blockermanschette 4 und des Kontrollballons 6 liegt die Rinne 14 frei, so daß die durch das Zuleitungsrohr 5 strömende Luft ohne weiteres in die Manschette 4 und in den Ballon 6 eintreten und diese füllen kann. Diese erfindungsgemäße Konstruktion, in der die Blockermanschette 4 und der Kontrollballon 6 als dünnwandige, aufgeweitete Teile eines sonst dicht an der Außenwand des Katheterrohres 1 anliegenden Folienschlauches 15 einstückig ausgebildet sind, bietet besondere fertigungstechnische Vorteile. Der Folienschlauch muß natürlich so gas- und flüssigkeitsdicht sein, daß er seine Funktion erfüllen kann.

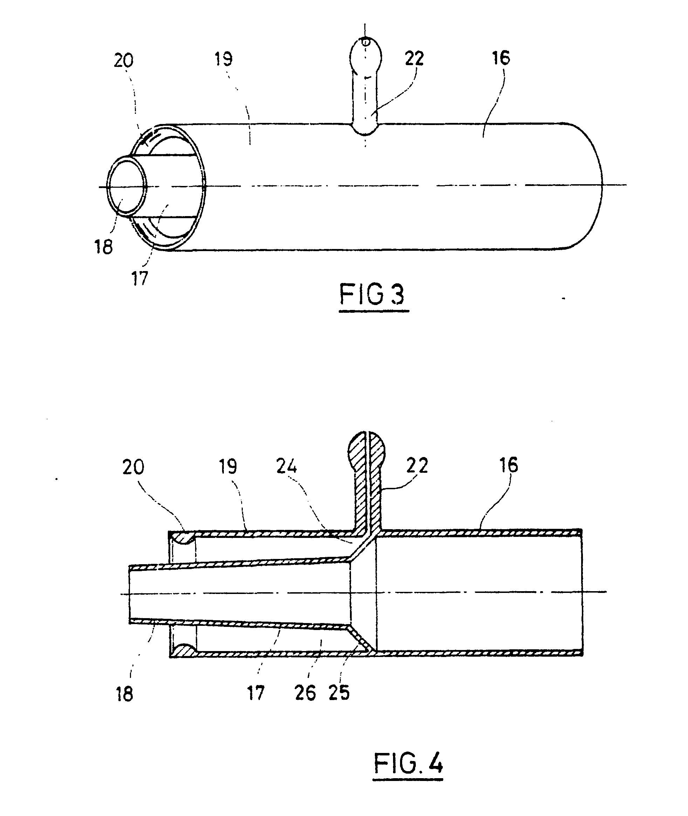

Das proximale Ende 3 des Endotrachealkatheters ist mit einem entsprechend angepaßten Konnektor 11 verbindbar. Solche angepaßten Konnektoren sind in den Figuren 3 bis 6 dargestellt. Wie aus den Figuren 3 un 4 zu ersehen ist, besteht der Konnektor aus einem weiten Rohrstutzen 16, einem Ansatzrohrstück 22 für die Luftzufuhrleitung, der äußeren Fortsetzung des weiten Rohrstutzens 16, hier als weites Rohrstück 19 bezeichnet, un einem engen Rohrstutzen 17, der konzentrisch innerhalb des weiten Rohrstücks 19 angeordnet ist.

Die spezifische Konstruktion des Konnektors geht im einzelnen aus Fig. 4 hervor. Der weite Rohrstutzen 16 dient zum Anschluß an das gewünschte medizinische Gerät, z. B. ein Beatmungs-, Narkose- oder Absauggerät, während der enge Rohrstutzen 17 für den Anschluß an das proximale Ende des Katheterrohres vorgesehen ist. Der weite Rohrstutzen 16 geht im Mittelstück des Konnektors in ein konisch sich verengendes Rohrstück 25 über, an das sich der enge Rohrstutzen 17 unmittelbar anschließt. Das weite Rohrstück 19 ist mit einem Ansatzrohrstück 22 versehen, an das eine Luftzufuhrleitung angeschlossen werden kann und das mit dem hohlzylinderförmigen Raum 26 über einen ringförmigen Durchlaß 24 verbunden ist.

Wenn das proximale Ende des Katheterrohres 1 auf den Rohrstutzen 17 aufgeschoben wird, bildet sich infolge der Anpassung des Konnektors an die Wandstärke des Katheterrohres eine gasdichte Abdichtung des eingeschobenen Katheterrohres gegenüber der Außenluft. Vorzugsweise ist der mittlere Abstand zwischen Innenwand des weiten Rohrstückes 19 und Außenwand des engen Rohrstutzens 17 gleich der Wandstärke des einzuführenden Katheterrohres, wobei geringfügige Abweichungen des mittleren Abstandes von der Wandstärke durchaus toleriert werden können.

Dadurch, daß das eingeführte proximale Ende des Katheterrohres den hohlzylinderförmigen Raum 26 von der Außenluft absperrt, hat der durch das Ansatzrohrstück 22 und den ringförmigen Durchlaß 24 austretende Luftstrom nur die Möglichkeit, durch die eine oder mehreren Anschlußöffnungen im Endstück des Katheterrohres in die entsprechenden Zuleitungsrohre einzutreten, von wo der Luftstrom in den Kontrollballon und in die Blockermanschette gelangt, die dadurch aufgeblasen werden.

Aufgrund dieser Konstruktion spielt es keine Rolle, in welcher Stellung das Katheterrohr mit dem Konnektor verbunden wird, weil in jeder beliebigen Stellung eine Verbindung zwischen den Anschlußöffnungen der Zuleitungsrohre im proximalen Ende des Katheterrohres und dem in den hohlzylindrischen Raum 26 eingeführten Luftstrom besteht.

Um das Aufschieben des Katheterrohres auf den Rohrstutzen 17 zu erleichtern und einen dichten Sitz auf dem Rohrstutzen zu gewährleisten, ist der enge Rohrstutzen 17 in einer bevorzugten Ausführungsform in Richtung auf das Endstück 18 konisch verjüngt.

Desgleichen ist vorzugsweise der weite Rohrstutzen 16 in Richtung auf das Mittelstück des Konnektors konisch verbreitert ausgebildet, wodurch ein fester und dichter Sitz des Anschlußrohres für das entsprechende medizinische Gerät noch besser gewährleistet wird.

Das offene Endstück 18 des engen Rohrstutzens 17 kann in einer Ebene mit dem Ende des weiten Rohrstückes 19 abschließen, es kann auch bereits innerhalb des weiten Rohrstückes 19 liegen, vorzugsweise ragt es jedoch über das Ende des weiten Rohrstückes 4 hinaus.

Das rasche, feste und gasdichte Aufstecken des proximalen Endes des Katheterrohres auf den engen Rohrstutzen 17 des Konnektors läßt sich noch dadurch verbessern, daß das offene Endstück des weiten Rohrstückes 19 mit einem ringförmigen Innenwulst oder Innengewinde 20 versehen ist. Dadurch wird das aufgeschobene Katheterrohr fest an die Außenwand des engen Rohrstutzens 17 angepreßt und eine vollständige Abdichtung nach außen erreicht.

Weitere vorteilhafte Ausführungsformen des angepaßten Konnektors ergeben sich durch Abwandlung des Ansatzrohrstückes 22. Dieses dient in der Regel zum Anschließen eines flexiblen Ansatzstückes, passend für Luftzufuhr- oder Blockerspritze, z. B. einer Luer-Spritze. Zu diesem Zwecke weist das Ansatzrohrstück 22 zweckmäßig eine an eine Blockerspritze angepaßte trichterförmige Öffnung auf. Ferner kann das Ansatzrohrstück 22 mit einem Rückschlagventil ausgerüstet sein. Hierdurch wird sichergestellt, daß die für einen bestimmten Innendruck in der Blockermanschette erforderliche eindosierte Luftmenge tatsächlich in dem System verbleibt und nicht etwa über das Ansatzrohrstück 22 entweichen kann.

In einer weiteren Ausgestaltung kann der Konnektor zwecks besserer Handhabbarkeit etwa in Höhe des Ansatzrohrstückes 22 auf der Außenwand des weiten Rohrstückes 19 einen Griffwulst oder ein oder mehrere Grifflaschen aufweisen.

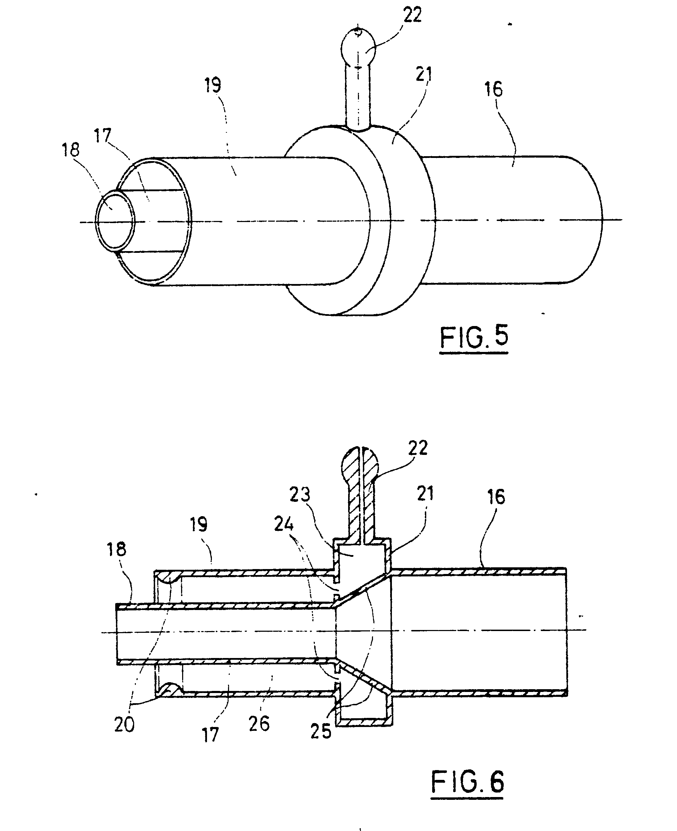

Weitere Ausführungsformen des angepaßten Konnektors sind in den Figuren 5 und 6 dargestellt. Der Konnektor besteht auch hier aus einem weiten Rohrstutzen 16, einem sich konisch verengenden Rohrstück 25 und einem engen Rohrstutzen 17 sowie einem den engen Rohrstutzen 17 konzentrisch umgebenden weiten Rohrstück 19 mit Innenrandwulst 20 am Ende des weiten Rohrstückes 19. Das Ansatzrohrstück 22 für die Luftzufuhrleitung befindet sich jedoch an einem als Ringstück 21 ausgebildeten Mittelstück des Konnektors über dem konisch sich verengenden Rohrstück 25.

Das Ringstück 21 kann massiv oder vorzugsweise hohl sein. Wenn es massiv ist, enthält es eine in den Figuren nicht dargestellte Rohrleitung oder Bohrung, die die Verbindung für den Luftstrom vom Ansatzrohrstück 22 zum hohlzylinderförmigen Raum 26 bildet. In der in Figur 6 dargestellten Ausführungsform ist das Ringstück 21 als Hohlkörper ausgebildet, so daß der Luftstrom durch das Ansatzrohrstück 22 in den ringförmigen Hohlraum 23 eintreten und durch ein oder mehrere Öffnungen 24 wieder austreten und in den hohlzylinderförmigen Raum 26 einströmen kann. Wie aus Fig. 6 ersichtlich ist, enthält das Ringstück 21 dementsprechend einen ringförmigen Hohlraum 23, der mit dem Ansatzrohrstück 22 direkt und über ein oder mehrere Öffnungen oder einen ringförmigen Durchlaß 24 mit dem hohlzylinderförmigen Raum 26 verbunden ist.

Der an den erfindungsgemäßen Ballonkatheter angepaßte Konnektor besteht aus einem sterilisierbaren Material. Für den wiederholten Gebrauch wird ein dauerhaftes Material verwendet, z. B. geeignete Metalle, wie V2A-Stahl, während für den Einmalgebrauch preiswertere Materialien, insbesondere bestimmte Kunststoffe, wie z. B. Polyolefine, insbesondere Polyethylen, Polyethylenvinylacetat (EVA), Polyvinylchlorid, Polystyrol sowie Copolymere wie SBS und ABS oder auch thermoplastische Polyurethane, eingesetzt werden.

Es ist möglich, die erfindungsgemäßen Ballonkatheter sowohl mit Niederdruckmanschette als auch mit Hochdruckmanschette auszurüsten.

Es versteht sich, daß die erfindungsgemäßen Ballonkatheter zwar insbesondere für den Einsatz als Endotrachealkatheter vorgesehen sind, daß sie aber aufgrund des universellen Aufbaus in zweckmäßig abgeänderter Form auch leicht für andere Zwecke, z. B. als Herz- und Blasenkatheter oder Magensonden, vorteilhaft angewendet werden können.

**Patentansprüche**

1. Ballonkatheter, insbesondere Endotrachealkatheter, mit einem Katheterrohr (1) aus elastischem, gewebefreundlichem Material mit einem offenen proximalen Ende (3), das mit einem angepaßten Konnektor (11) verbindbar ist, einer in der Nähe des distalen Rohrendes (2) auf dem Katheterrohr (1) angeordneten, mit ihm fest verbundenen, aufblasbaren Blockermanschette (4), einem in der Nähe des proximalen Rohrendes (3) auf dem Katheterrohr (1) angeordneten, mit ihm fest verbundenen aufblasbaren Kontrollballon (6) und ein oder mehreren Zuleitungsrohren (5), die die Blockermanschette (4) mit dem Kontrollballon (6) und mit einem Ansatzstutzen (13) für eine Blockerspritze verbinden, dadurch gekennzeichnet, daß das Katheterrohr (1) mit einem abgeschrägten, offenen distalen Ende (2) über seine gesamte Länge in an sich bekannter Weise durch Aufextrudieren eines dünnwandigen Folienschlauches (15) ummantelt ist, wobei dieser Folienschlauch (15) in den für die Blockermanschette (4) und den Kontrollballon (6) vorgesehenen Bereichen lose auf der Außenwand des Katheterrohres (1) aufliegt, während der Schlauch (15) in den übrigen Bereichen fest und dicht mit der Außenwand des Katheterrohres (1) verbunden ist, wobei das oder die Zuleitungsrohre (5), die sich über die gesamte Länge des Katheterrohres (1) erstrecken und am distalen Rohrende (2) dicht verschlossen sind, durch jeweils eine Rinne (14) in der Außenwand des Katheterrohres (1) und den das Katheterrohr (1) dicht ummantelnden, die Rinne (14) nach außen gas- und flüssigkeitsdicht abdeckenden Folienschlauch (15) gebildet sind, und daß das Katheterrohr (1) und das bzw. die Zuleitungsrohre (5) am proximalen Ende (3) mit dem Konnektor (11) über eine oder mehrere Anschlußöffnungen (10) und entsprechende Ansatzstutzen (12, 13) verbindbar sind.

2. Katheter nach Anspruch 1, dadurch gekennzeichnet, daß das Katheterrohr (1) und der Folienschlauch (15), aus dem an den vorgesehenen Bereichen Blockermanschette (4) und Kontrollballon (6) gebildet werden, aus einem synthetischen Polymeren, aus Gummi oder aus hochpolymeren elastischen Organo-Silicium-Verbindungen bestehen.

3. Katheter nach Anspruch 2, dadurch gekennzeichnet, daß das Katheterrohr (1) und der Folienschlauch (15) aus PVC, Hochdruckpolyethylen oder einem thermoplastischen Polyurethan bestehen.

4. Katheter nach den Ansprüchen 1 bis 3, dadurch gekennzeichnet, daß der Folienschlauch (15) außer an den für die Blockermanschette (4) und den Kontrollballon (6) vorgesehenen Bereichen mit dem Katheterrohr durch Verkleben, Siegeln, Verschweißen und/oder Aufschrumpfen fest und gas- und flüssigkeitsdicht verbunden ist.

5. Katheter nach den Ansprüchen 1 bis 4, dadurch gekennzeichnet, daß der Folienschlauch (15) in den für die Blockermanschette (4) und den Kontrollballon(6) vorgesehenen Bereichen aufgeweitet und/oder zu einer Blase gewünschter Form und Größe ausgeformt ist.

6. Katheter nach den Ansprüchen 1 bis 5, dadurch gekennzeichnet, daß er aus einem als Endlosrohr durch ein an sich bekanntes kombiniertes Schlauchextrusions- und Extrusionsblasverfahren kontinuierlich erzeugten und in bestimmten Abständen abgelängten ummantelten Katheterrohr hergestellt worden ist.

7. Katheter nach den Ansprüchen 1 bis 6, dadurch gekennzeichnet, daß er mit einem an den Katheter angepaßten Konnektor (11) versehen ist, der einen weiten Rohrstutzen (16) und einen engen Rohrstutzen (17) aufweist, wobei der Übergang von dem weiten Rohrstutzen (16) zu dem engen Rohrstutzen (17) durch ein konisch geformtes Rohrstück (25) gebildet wird, und ein weites Rohrstück (19) enthält, das um den engen Rohrstutzen (17) konzentrisch angeordnet und mit einem Ansatzrohrstück (22) für eine Luftzufuhrleitung versehen ist, wobei das Ansatzrohrstück (22) über einen ringförmigen Durchlaß (24) mit dem zwischen der Innenwand des weiten Rohrstückes (19) und der Außenwand des engen Rohrstutzens (17) gebildeten hohlzylinderförmigen Raum (26) verbunden ist und der Abstand zwischen der Innenwand des weiten Rohrstückes (19) und der Außenwand des engen Rohrstutzens (17) der Wandstärke des einzuführenden Katheterrohres (1) so angepaßt ist, daß sich eine gasdichte Verbindung ergibt.

8. Katheter nach den Ansprüchen 1 bis 7, dadurch gekennzeichnet, daß der enge Rohrstutzen (17) des Konnektors (11) in Richtung auf das Endstück (18) konisch verjüngt ist.

9. Katheter nach den Ansprüchen 1 bis 8, dadurch gekennzeichnet, daß der weite Rohrstutzen (16) des Konnektors (11) in Richtung auf das Mittelstück des Konnektors (11) konisch verbreitert ausgebildet ist.

10. Katheter nach einem der Ansprüche 1 bis 9, dadurch gekennzeichnet, daß das offene Endstück (18) des engen Rohrstutzens (17) des Konnektors (11) über das Ende des weiten Rohrstückes (19) hinausragt.

11. Katheter nach einem der Ansprüche 1 bis 10, dadurch gekennzeichnet, daß das offene Endstück des weiten Rohrstückes (19) des Konnektors (11) mit einem ringförmigen Innenwulst (20) versehen ist.

12. Katheter nach einem der Ansprüche 1 bis 10, dadurch gekennzeichnet, daß das offene Endstück des weiten Rohrstückes (19) des Konnektors (11) mit einem Innengewinde versehen ist.

**Claims**

1. A balloon catheter, particularly an endotracheal catheter, having a catheter pipe (1) of elastic, tissue-compatible material with an open proximal end (3), which can be connected to a matched connector (11); an inflatable blocker sleeve (4) located on the catheter pipe (1) near the distal end of the pipe (2) and firmly connected to it; an inflatable control balloon (6) located on the catheter pipe (1) near the proximal end of the tube (3) and firmly connected to it; and one or more feed pipes (5), which connect the blocker sleeve (4) to the control balloon (6) and to the adjoining connection (13) for a blocker injector;

characterised in that the catheter pipe (1) with a canted, open distal end (2) is encased by extruding-on a thin-walled film-like tube (15) along its entire length in a manner known per se, whereby this film-like tube (15) lies loosely on the outer wall of the catheter pipe (1) in the regions provided for the blocker sleeve (4) and the control balloon (6), while the tubing (15) is connected in the remaining regions in such a way that is fixed and sealed to the outer wall of the catheter pipe (1), whereby the feed pipe, or pipes, (5) which run along the entire length of the catheter pipe (1) and are sealed tight at the distal end (2) of the pipe, are each formed by a channel (14) in the outer wall of the catheter pipe (1) and the film-like tube (15) which closely encases the catheter pipe (1), and covers the channel (14) so that it is gas and liquid-tight to the outside, and in that the catheter pipe (1) and the feed pipe, or pipes, (5) can be connected at the proximal end (3) with the connector (11) via one or more connecting openings (10) and corresponding adjoining connections (12, 13).

2. Catheter according to claim 1, characterised in that the catheter pipe (1) and the film-like tube (15), from which the blocker sleeve (4) and control balloon (6) are formed at the regions provided, are made of a synthetic polymer, of rubber, or of high-polymer elastic organo-silicium compounds.

3. Catheter according to claim 2, characterised in that the catheter pipe (1) and the film-like tube (15) are made of PVC, high-pressure polyethylene or a thermoplastic polyurethane.

4. Catheter according to claims 1 to 3, characterised in that the film-like tube (15) is connected to the catheter pipe by bonding, sealing, welding and/or shrinking-on so that it is firmly connected and gas and liquid-tight, apart from at the regions provided for the blocker sleeve (4) and the control balloon (6).

5. Catheter according to claims 1 to 4, characterised in that the film-like tube (15) is widened in the regions provided for the blocker sleeve (4) and the control balloon (6) and/or is shaped into a blister of the desired shape and size.

6. Catheter according to claims 1 to 5, characterised in that it has been manufactured as an encased tube continuously produced as an endless tube by a combined tube-extrusion and extrusion-blister procedure which is known per se, and cut off in specified lengths.

7. Catheter according to claims 1 to 6, characterised in that it is provided with a connector (11) which is matched with this catheter and which has a wide pipe connection (16) and a narrow pipe connection (17), whereby the junction from the wide pipe connection (16) to the narrow pipe connection (17) is formed by a conical section of pipe, and contains a wide section of pipe (19), which is arranged concentrically around the narrow pipe connection (17) and is provided with an adjoining section of pipe (22) for an air feed pipe, whereby the adjoining section of pipe (22) is connected via an annular passage (24) to the space (26) shaped as a hollow cylinder which is formed between the inner wall of the wide section of pipe (19) and the outer wall of the narrow section of pipe (17), and the distance between the inner wall of the wide section of pipe (19) and the outer wall of the narrow section of pipe (17) is matched to the wall thickness of the catheter pipe (10) so that a gas-tight connection results.

8. Catheter according to the claims 1 to 7, characterised in that the narrow pipe connection (17) of the connector (11) is tapered conically in the direction of the end section (18).

9. Catheter according to the claims 1 to 8, characterised in that the wide pipe connection (16) of the connector (11) is formed so that it widens conically in the direction of the centre piece of the connector (11).

10. Catheter according to one of the claims 1 to 9, characterised in that the open end section (18) of the narrow pipe connection (17) of the connector (11) projects beyond the end of the wide section of pipe (19).

11. Catheter according to one of the claims 1 to 10, characterised in that the open end piece of the wide section of pipe (19) of the connector (11) is provided with an annular internal bead (20).

12. Catheter according to one of the claims 1 to 10, characterised in that the open end piece of the wide section, of pipe (19) of the connector (11) is provided with an internal thread.

**Revendications**

1. Cathéter à ballonnet, notamment cathéter endotrachéal, avec un tube de cathéter (1) en un matériau élastique, acceptable pour les tissus, comportant une extrémité proximale ouverte (3), que l'on peut raccorder à un connecteur approprié (11), un manchon d'arrêt (4) gonflable, agencé sur le tube de cathéter (1) à proximité de l'extrémité distale (2) du tube et fermement assujetti à ce tube, un ballonnet de contrôle (6) gonflable, agencé sur le tube de cathéter (1) au voisinage de l'extrémité proximale (3) du tube et fermement assujetti à ce tube, et un ou plusieurs tubes d'amenée (5) qui relient le manchon d'arrêt (4) au ballonnet de contrôle (6) et à une pièce de raccord (13) pour une seringue d'arrêt, caractérisé en ce que le tube de cathéter (1) avec une extrémité distale ouverte, en biseau (2), est enveloppé par extrudage, d'une manière en soi connue, sur toute sa longueur, d'un tuyau en forme de feuille à paroi mince (15), où ce tuyau en forme de feuille (15) repose de manière lâche sur la paroi externe du tube de cathéter (1) dans les zones prévues pour le manchon d'arrêt (4) et le ballonnet de contrôle (6), cependant que le tuyau (15) est relié fermement et hermétiquement à la paroi externe du tube de cathéter (1) dans les autres zones, où le ou les tubes d'amenée (5) qui s'étendent sur toute la longeur du tube de cathéter (1) et qui sont fermés de façon étanche à l'extrémité (2) distale du tube, sont formés chaque fois par un canal (14) dans la paroi externe du tube de cathéter (1), et le tuyau en forme de feuille (15) enveloppant hermétiquement le tube de cathéter (1), isolant le canal (14) vers l'extérieur, de façon étanche aux gaz et aux liquides, et en ce que le tube de cathéter (1) et le ou les tubes d'amenée (5) à l'extrémité proximale (3) peuvent être raccordés au connecteur (11) par l'intermédiaire d'une ou de plusieurs ouvertures de raccordement (10) et des pièces de raccord correspondantes (12, 13).

2. Cathéter suivant la revendication 1, caractérisée en ce que le tube de cathéter (1) et le tuyau en forme de feuille (15), à partir duquel sont formés le manchon d'arrêt (4) et le ballonnet de contrôle (6) dans les zones prévues, se composent d'un polymère de synthèse, de caoutchouc ou de composés d'organosilicium, élastiques, fortement polymères.

3. Cathéter suivant la revendication 2, caractérisée en ce que le tube de cathéter (1) et le tuyau en forme de feuille (15) se composent de PVC, de polyéthylène haute pression ou d'un polyuréthane thermoplastique.

4. Cathéter suivant les revendications 1 à 3, caractérisée en ce que le tuyau en forme de feuille (15) est relié ou raccordé, fermement et de manière étanche aux gaze et aux liquides, au tube de cathéter sauf dans les zones prévues pour le manchon d'arrêt (4) et de ballonnet de contrôle (6), par collage, scellage, soudure et/ou serrage par contraction ou retrait.

5. Cathéter suivant les revendications 1 à 4, caractérisée en ce que le tuyau en forme de feuille (15) est élargi et/ou est conformé en une bulle de grandeur et de forme souhaitées dans les zones prévues pour le manchon d'arrêt (4) et le ballonnet de contrôle (6).

6. Cathéter suivant les revendications 1 à 5, caractérisée en ce qu'elle a été fabriquée à partir d'un tube de cathéter sans fin continuellement produit par un procédé en soi connu d'extrusion de tuyau et d'extrusion par soufflage combiné et enveloppé à des distances déterminées et sur des longueurs voulues.

7. Cathéter suivant les revendications 1 à 6, caractérisée en ce qu'elle est pourvue d'un connecteur (11) adapté au cathéter, lequel connecteur présente une tubulure large (16) et une tubulure étroite (17), où la transition de la tubulure large (16) à la tubulure étroite (17) est formée par une pièce tubulaire conique (25) et contient une pièce tubulaire large (19), concentriquement agencée autour de la tubulure étroite (17) et pourvue d'un appendice tubulaire (22) pour une conduite d'amenée d'air, où l'appendice tubulaire (22) est mis en communication par l'intermédiaire d'un passage annulaire (24), avec l'espace cylindrique creux (26) formé entre la paroi interne de la pièce tubulaire large (19) et la paroi externe de la tubulure étroite (17) et la distance entre la paroi interne de la pièce tubulaire (19) et la paroi externe de la tubulure étroite (17) est ainsi adaptée à l'épaisseur de la paroi du tube de cathéter (1) à introduire, qu'un raccord ou une connexion étanche aux gaz s'établit.

8. Cathéter suivant les revendications 1 à 7, caractérisée en ce que la tubulure étroite (17) du connecteur (11) s'effile coniquement en direction de la pièce d'extrémité (18).

9. Cathéter suivant les revendications 1 à 8, caractérisée en ce que la tubulure large (16) du connecteur (11) s'évase coniquement en direction de la pièce médiane du connecteur (11).

10. Cathéter suivant l'une des revendications 1 à 9, caractérisée en ce que la pièce d'extrémité (18) ouverte de la tubulure étroite (17) du connecteur (11) s'étend au-delà de l'extrémité de la pièce tubulaire large (19).

11. Cathéter suivant l'une des revendications 1 à 10, caractérisée en ce que la pièce d'extrémité ouverte de la pièce tubulaire large (19) du connecteur (11) est pourvue d'un renflement ou bourrelet (20).

12. Cathéter suivant l'une des revendications 1 à 10, caractérisée en ce que la pièce d'extrémité ouverte de la pièce tubulaire large (19) du connecteur (11) est pourvue d'un file interne.

Fig.1

Fig.2

1

FIG 3

FIG. 4

FIG.5

FIG.6